# EUROPEAN PATENT APPLICATION

(11) **EP 3 633 367 A1**
(43) Date of publication of application: **08.04.2020**
(21) Application number: 18198131.7
(22) Date of filing: 02.10.2018
(51) Int. Cl.: G01N 33/00, G01N 33/14

(54) **DIGITAL SYSTEM FOR MEASURING AND CONTROLLING ORGANOLEPTIC PARAMETERS IN CRAFT BEER PRODUCTION**

(71) Applicant: Hesperia Societa' Cooperativa Sociale a r.l., 88100 Catanzaro (CZ) (IT)
(72) Inventor: RICCI, Luciano, 88100 CATANZARO (CZ) (IT)
(74) Representative: Scorza, Federica

(57) **Abstract**

A digital system (100) for measuring and controlling organoleptic parameters in craft beer production comprises: a plurality of biosensors (101) configured to evaluate organoleptic parameters of the beer; a plurality of electronic control boards (102) each of them being connected to its own biosensor (101); a plurality of expansion electronic boards (105) each of them being connected to its own electronic control board (102); a temperature probe (107) measuring the working conditions of the plurality of biosensors (101); an electronic board (103). The digital system (100), in addition, comprises a module (106) including a Peltier cell, and an electronic system (104) for the regulation of physical parameters according to a PID control connected to another expansion control board (105d), to the temperature probe (107) and to the module (106).

## Description

The present invention relates to a digital system for measuring and controlling organoleptic parameters in craft beer production.

In particular, the present invention relates to a digital system for measuring and controlling organoleptic parameters in craft beer production, of the type comprising artificial biosensors and electronic control systems.

In the production of the beer, the handcrafted product distinguishes from the industrial one for a series of features of different kind: variety, organoleptic features and nutritional supply. These features of the handcrafted beers are highly appreciated by consumers, who are willing to pay a price even five times higher than that of an industrial beer.

The main limit in the production of a craft beer comes with the effort to recreate successful recipes as identical as possible in time, considering that the control tools the artisan owns and the external elements that may influence the final product depend largely on the personal skills of the artisan himself to manage the system, without having an instrumental feedback that helps to avoid typical human errors (diseases of the ear-nose-throat apparatus, periods of different perceptions of tastes, flavours and smells during the year, variations of the basic components of the commodities, etc.)

The artisan, indeed, usually relies on his own senses to verify the main parameters of the process and the features of the product.

Digital systems for measuring and controlling organoleptic parameters characterized by the presence of artificial sensors for the organoleptic analysis of foods are currently known.

A first example is the patent CN103018293 which describes a system to detect parameters concerning the four basic flavours (acid, bitter, sweet and salty) in liquid foods, using a biosensor that acts as an electronic "tongue". The system comprises the following phases: preparation of a solution of HCl with a concentration of 0,01 mol/l from distilled water as the activating solution of the sensor acting as an electronic tongue; use of distilled water of the cleaning solution; addition of HCl solution as the calibration solution, so as to use the electronic sensor of the tongue to analyze the samples after the calibration; selection of four types of substances having four basic flavours with a concentration suitable to the typical concentration level inside the human mouth as basic samples; determination of the number of cleaning glasses based upon the number of the samples, where each sample is cleaned inside the specific cleaning becher and the samples are cleaned according to the natural sequence; positioning of the electronic sensor of the tongue in each becher for 120 seconds to detect the flavour of the sample making six measures for each sample to assure that the flavor of the sample has a semi-logarithmic relation with the reaction force on the electronic sensor of the tongue; implementation of macro software on the results to get the relation of relative intensity between different concentrations of the basic substances on the electronic tongue.

The patent CN106645766, otherwise, describes a system for the smart detection of the flavor of bovine meat. The detection system comprises an automatic device for the analysis of the quality of the flavor, a control system based on the use of a PLC, a data acquisition and analysis system and a driving system. The automatic device for the detection of the quality of the beef comprises an external box, a machinery that conveys the matrix of the sensors, a sliding table with a linear guide with spheres, a mobile table with a horizontal sample and a positioning system. The data acquisition and analysis system comprise a sensor array and a data acquisition board; the driving system comprises a first stepper motor, a second stepper motor, an electric push rod, a reducer and a calculator. According to the specifications of the system the PLC acts as a control core and the taste quality levels of the meats are automatically measured.

However, the known digital systems for measuring and controlling organoleptic parameters like those described suffer limitations concerning the low applicability to the production of craft beer and the difficulty to maintain fixed the reference values of organoleptic parameters such as color, bitter grade (IBU) and smell.

The purpose of the present invention is to provide a digital system for measuring and controlling organoleptic parameters in the craft beer production aimed at assuring the uniformity of the original recipe of the product, fixing the deviations of fundamental qualitative indicators, thus having characteristics that exceeds the limits that still affect the current systems for measuring and controlling organoleptic parameters in craft beer production, with reference to the known technique.

According to the present invention, a digital system for measuring and controlling organoleptic parameters in the craft beer production is provided, as defined in claim 1.

For a better understanding of the present invention it is now described a preferred embodiment, purely by way of a non-limiting example, with reference to the accompanying drawings, in which:

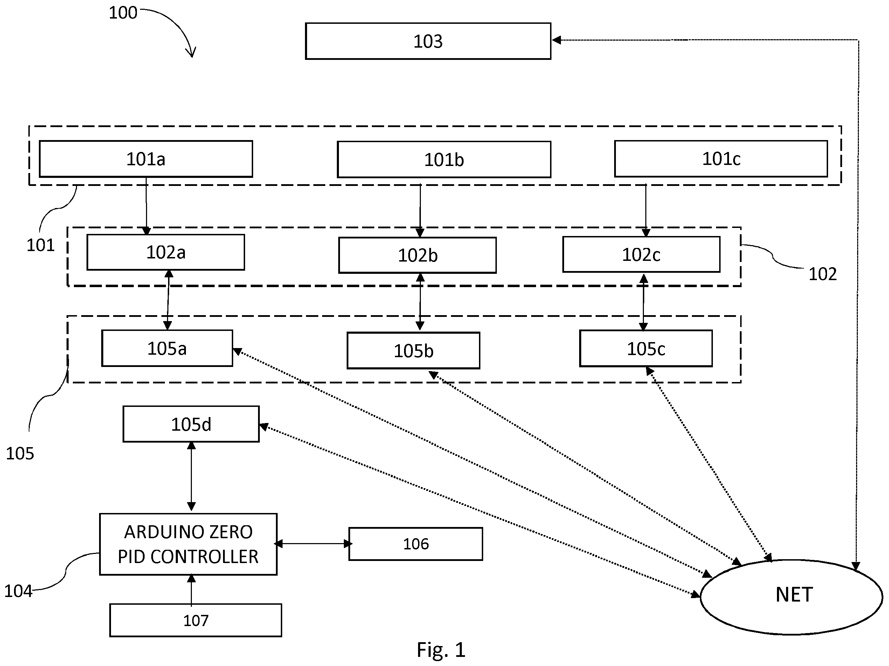

- figure 1 shows a block diagram of a digital system for measuring and controlling organoleptic parameters in the craft beer production, according to the invention.

With reference to this figure, a digital system for measuring and controlling organoleptic parameters in the craft beer production is shown, according to the invention.

In particular, the digital system 100 for measuring and controlling organoleptic parameters in the craft beer production comprises:
- a plurality of biosensors 101 configured to evaluate organoleptic parameters of the beer;
- a plurality of electronic control boards 102 each of them being connected to its own biosensor 101;
- a plurality of expansion electronic boards 105, each of them being connected to its own electronic control board 102;
- a temperature probe 107 measuring the working conditions of the plurality of biosensors 101;
- an electronic board 103;
- a module 106 including a Peltier cell, and an electronic system 104 for the regulation of physical parameters according to a PID control connected to another expansion control board 105d, to the temperature probe 107 and to the module 106.

According to the preferred embodiment shown in figure 1, the plurality of biosensors 101 comprises at least three biosensors.

According to an aspect of the invention, the biosensors 101 are chosen among:
- potentiometric membranes;
- sensors for spectrophotometry;
- sensors for gas detection.

According to an aspect of the invention, the plurality of biosensors 101 comprises a first biosensor 101a, that in use acts as an electronic nose, configured to measure the smell of craft beer, composed of an array of sensors for the detection of LPG (Liquefied Petroleum Gas). The array of sensors is made of aluminum oxide ceramic tubes, having a tin dioxide sensible layer.

According to an aspect of the invention, the plurality of biosensors 101 comprises a second biosensor 101b, made of a potentiometric membrane that in use acts as an electronic tongue, configured to measure the bitter grade (IBU) of craft beer.

According to an aspect of the invention, the plurality of biosensors 101 comprises a third biosensor 101c, that in use acts as an electronic eye, configured to measure the color of craft beer, comprising an array of photodiodes, produced by CMOS technology.

As shown in figure 1, the three biosensors 101a, 101b e 101c are connected respectively to three electronic boards 102a, 102b and 102c comprised in the plurality of electronic control boards 102.

Advantageously according to the invention, the electronic system 104 powers and controls the electric polarity, through PWM (Pulse Width Modulation), of the module 106 with Peltier cell configured to maintain constant and fixed the temperature of an analyzed craft beer sample, so as to guarantee the accuracy of the measure of the parameters monitored by the plurality of biosensors 101.

The electronic system 104, in use, measure the deviation of the thermal parameters of the samples of craft beer picked up from a main tank and submitted for analysis, being the organoleptic parameters detected by the biosensors 101 to measure at a fixed temperature, preferably 25°C, according to the electric specifications reported on the datasheets of the producers of the same biosensors 101.

According to another aspect of the invention, each electronic control board 102 is connected to an electronic expansion board 105 configured to carry out the connection to the local network and to the world wide web.

According to an aspect of the invention, each electronic control board 102 is provided with a web server configured to allow the visualization of the parameters measured by the biosensors 101a, 101b e 101c and the fixing of the reference parameters of the electronic system 104. For example, speaking of the third biosensor 101c it is possible to visualize the frequencies related to the three fundamental colors (R=red, B=blue, G=green) and white (W), as well as the color coming from the comparison with the values read through the EBC scale.

According to another aspect of the invention, the third biosensor 101c is inserted into a hermetically sealed case having a white inner surface, so as to avoid infiltrations of external light which would affect the accuracy of the measurement.

The present invention also concerns a device including a plurality of biosensors 101 configured to evaluate organoleptic parameters of the beer, a plurality of electronic control boards 102 each of them being connected to its own biosensor 101, a plurality of expansion electronic boards 105, each of them being connected to its own electronic control board 102, a temperature probe 107 measuring the working conditions of the plurality of biosensors 101, an electronic board 103, a module 106 including a Peltier cell, and an electronic system 104 for the regulation of physical parameters according to a PID control connected to another expansion control board 105d, to the temperature probe 107 and to the module 106.

The device in use measures and controls the organoleptic parameters of a sample of craft beer picked up from a main tank.

Advantageously according to the invention, the digital system 100 allows to monitor and control the fundamental organoleptic parameters in a process of production of craft beer, by means of a simple, automatic and affordable instrumentation.

A further advantage of the digital system 100 is that the plurality of electronic control boards 102 and the electronic board 103 are programmable under a multiplatform (LINUX, iOS, MS Windows) open source environment.

Finally, the digital system 100 is inexpensive.

Finally, it is clear that the digital system for measuring and controlling organoleptic parameters in craft beer production described and illustrated here, may be modified and varied without thereby departing from the scope of the present invention, as defined in the appended claims.

## Claims

1. Digital system (100) for measuring and controlling organoleptic parameters in craft beer production comprising:
- a plurality of biosensors (101) configured to evaluate organoleptic parameters of the beer;
- a plurality of electronic control boards (102) each of them being connected to its own biosensor (101) ;
- a plurality of expansion electronic boards (105) each of them being connected to its own electronic control board (102);
- a temperature probe (107) measuring the working conditions of the plurality of biosensors (101);
- an electronic board (103);
**characterized in that** it comprises a module (106) including a Peltier cell, and an electronic system (104) for the regulation of physical parameters according to a PID control connected to another expansion control board (105d), to the temperature probe (107) and to the module (106) .

2. Digital system (100) for measuring and controlling organoleptic parameters in craft beer production according to claim 1, **characterized in that** said plurality of biosensors (101) comprises at least a first biosensor (101a) configured to measure the smell of craft beer, a second biosensor (101b), configured to measure the bitter grade (IBU) of the craft beer, and a third biosensor (101c) configured to measure the color of the craft beer.

3. Digital system (100) for measuring and controlling organoleptic parameters in craft beer production according to claim 2, **characterized in that** said first biosensor (101a) is configured to measure the presence of LPG (Liquefied Petroleum Gas) and is composed of an array of sensors made of aluminum oxide ceramic tubes, having a tin dioxide sensible layer.

4. Digital system (100) for measuring and controlling organoleptic parameters in craft beer production according to claim 2, **characterized in that** said second biosensor (101b) is made of a potentiometric membrane.

5. Digital system (100) for measuring and controlling organoleptic parameters in craft beer production according to claim 2, **characterized in that** said third biosensor (101c) comprises an array of photodiodes and is produced by CMOS technology.

6. Digital system (100) for measuring and controlling organoleptic parameters in craft beer production according to claim 2, **characterized in that** the third biosensor (101c) is inserted into a hermetically sealed case having a white inner surface, so as to avoid infiltrations of external light.

7. Digital system (100) for measuring and controlling organoleptic parameters in craft beer production according to claim 1, **characterized in that** said electronic system (104) powers and controls the electric polarity, through a PWM (Pulse Width Modulation) logic, of the module (106) with a Peltier cell configured to maintain a constant temperature of the sample of craft beer under analysis.

8. Digital system (100) for measuring and controlling organoleptic parameters in craft beer production according to claim 1, **characterized in that** each electronic control board (102) is connected to its own expansion electronic board (105) configured to carry out the connection to the local network and to the world wide web.

9. Digital system (100) for measuring and controlling organoleptic parameters in craft beer production according to claim 1, **characterized in that** each electronic control board (102) is provided with a web-server configured to allow a visualization of the parameters measured by the biosensors (101a, 101b, 101c) and to set reference parameters of the electronic system (104) .
